# EUROPÄISCHE PATENTSCHRIFT

(11) **EP 1 022 012 B1**
(45) Veröffentlichungstag und Bekanntmachung des Hinweises auf die Patenterteilung: **23.11.2005**
(21) Anmeldenummer: 00250006.4
(22) Anmeldetag: 11.01.2000
(51) Int. Cl.: A61K 6/093

(54) **Dentalmaterialien auf der Basis von Polysiloxanen**
Polysiloxane based dental materials
Matériaux dentaires à base de polysiloxane

(30) Priorität: 21.01.1999 DE 19903177
(43) Veröffentlichungstag der Anmeldung: 26.07.2000
(73) Patentinhaber: Ivoclar Vivadent AG, 9494 Schaan (LI)
(72) Erfinder: Moszner, Norbert, Prof. Dr., 9495 Triesen (LI); Völkel, Thomas, Dr., 88131 Lindau (DE); Stein, Sabine, 6710 Nenzing (AT); Rheinberger, Volker, Dr., 9490 Vaduz (LI)
(74) Vertreter: UEXKÜLL & STOLBERG

(56) Entgegenhaltungen:
- EP-A- 0 261 520
- WO-A-98/28307
- DE-A- 3 407 087
- DE-A- 4 133 494
- DE-A- 19 619 046
- DE-A- 19 714 320
- DE-C- 4 416 857
- GB-A- 2 023 628

## Beschreibung

Die Erfindung betrifft Dentalmaterialien auf der Basis von polymerisationsfähigen, methacrylatmodifizierten Polysiloxanen.

Dentalmaterialien auf Basis polymerisationsfähiger Silane sind bekannt. Die DE 36 10 804 A1 offenbart Dentalharzmassen, die Siloxanpolymere, mit dem Siloxanpolymeren copolymerisierbare Monomere und einen Polymerisationskatalysator enthalten. Die Dentalharzmassen sollen nach der Polymerisation eine verbesserte Druckfestigkeit, Abriebbeständigkeit und Biegefestigkeit aufweisen.

Die DE 34 07 087 A1 und WO 92/16183 betreffen die Verwendung von Zusammensetzungen auf der Basis von organisch modifizierten Kieselsäurepolykondensaten zur Beschichtung von Zähnen und Zahnersatzteilen. Die gehärteten Überzüge sollen gegenüber der Anlagerung von Plaque resistent sein.

Aus der DE 41 33 494 sind Dentalharzmassen auf der Basis von polymerisierbaren Polysiloxanen bekannt, die durch hydrolytische Kondensation eines oder mehrerer Silane hergestellt werden, von denen mindestens eines durch einen 1,4,6-Trioxaspiro-[4,4]-nonan-Rest oder eine (Meth)acrylatgruppe substituiert ist, wobei letztere vorzugsweise eine Thioetherfunktion enthält. Die Dentalharzmassen sollen bei der Härtung nur eine geringe Volumenänderung zeigen, jedoch sind Silane mit Orthoestergruppen schwierig zugänglich und wenig lagerstabil während Thioethergruppen oxidationsempfindlich sind.

Die DE 196 19 046 offenbart schrumpfungsarme polymerisierbare Zusammensetzungen auf der Basis von Mercapto- oder Norbornensilanen und einem Reaktionspartner für die En-Thiol-Polymerisation. Die Härtung dieser Zusammensetzungen verläuft unter geringem Polymerisationsschrumpf und ergibt Produkte mit hoher mechanischen Festigkeit, die jedoch ebenfalls oxidationsempfindliche Thioethergruppen enthalten.

Aufgabe der Erfindung ist die Bereitstellung von Dentalmaterialien auf der Basis von Polysiloxanen, die sich in organischanorganische Verbundmaterialien kovalent einbauen lassen und keine Spiro- oder Thioethergruppen enthalten.

Die Aufgabe wird durch Dentalmaterialien gelöst, die mindestens ein Polysiloxan auf der Basis von einem oder mehreren Silanen gemäß der Formel (I)

[(W_{q}-R⁶-Z)ₚ-R³]ₘY-R²-SiXₙR¹ ₃₋ₙ Formel (I)

enthalten, in der
- X: für ein Halogenatom, eine Hydroxyl-, Alkoxy- und/oder Acyloxygruppe steht;
- n: gleich 1 bis 3 ist;
- R¹: für eine Alkyl-, Alkenyl-, Aryl-, Alkylaryl-, Arylalkylgruppe steht;
- R²: für eine Alkylengruppe steht;
- R³: für einen p-fach substituierten, geraden, verzweigten oder cyclischen, gesättigten oder ungesättigten, aromatischen oder aliphatischen organischen Rest mit 2 bis 40 Kohlenstoffatomen und ggf. 1 bis 6 Heteroatomen steht;
- R⁶: für einen q-fach substituierten, geraden, verzweigten oder cyclischen organischen Rest mit 1 bis 20 Kohlenstoffatomen steht ;
- p: gleich 1 bis 6 ist;
- q: gleich 1 bis 6 ist;
- Y: für -NR⁴-, N oder -(C=O)-NH- steht;
- m: gleich 2 für Y = N und gleich 1 für Y = -NR⁴- oder -(C=O)-NH-ist;
- R⁴: für eine Alkyl- oder Arylgruppe steht;
- Z: für O, S, -(C=O)-O-, -(C=O)-NH-, -O-(C=O)-NH- steht oder entfällt;
- W: für CH₂=CR⁵-(C=O)-O- steht; und
- R⁵: für ein Wasserstoffatom oder eine Alkylgruppe steht.

Geeignete Heteroatome sind Phosphor und vorzugsweise Sauerstoff.

In der gesamten Beschreibung sowie den Ansprüchen werden unter Alkyl-, Acyloxy-, Alkoxy-, Alkenylgruppen und Alkylengruppen Reste verstanden, die vorzugsweise 1 bis 25 Kohlenstoffatome, besonders bevorzugt 1 bis 10 Kohlenstoffatome und ganz besonders bevorzugt 1 bis 4 Kohlenstoffatome enthalten und gegebenenfalls einen oder mehrere Substituenten, wie zum Beispiel Halogenatome, Nitrogruppen oder Alkyloxyreste tragen. Mit Aryl sind Reste, Gruppen oder Substituenten gemeint, die vorzugsweise 6 bis 10 Kohlenstoffatome aufweisen und wie vorstehend angegeben substituiert sein können. Die obigen Definitionen gelten auch für zusammengesetzte Gruppen wie zum Beispiel Alkylaryl- und Arylalkylgruppen. Eine Alkylarylgruppe bezeichnet somit beispielsweise eine wie oben definierte Arylgruppe, die mit einer wie oben definierten Alkylgruppe substituiert ist.

Die Alkyl-, Acyloxy-, Alkoxy-, Alkenylgruppen und Alkylengruppen können geradkettig, verzweigt oder cyclisch sein.

Unabhängig voneinander wählbare bevorzugte Definitionen für die einzelnen Variablen sind:
- X =: eine Methoxy- und/oder Ethoxygruppe;
- n =: 2 oder 3;
- R¹ =: eine C₁- bis C₃-Alkylgruppe, insbesondere eine Methylgruppe;
- R² =: eine C₁- bis C₄-Alkylengruppe;
- R³ =: ein p-fach substituierter, gerader, verzweigter oder cyclischer, gesättigter oder ungesättigter, aromatischer oder aliphatischer organischer Rest mit 2 bis 10 Kohlenstoffatomen und ggf. einem Heteroatom, vorzugsweise einem Sauerstoffatom, besonders bevorzugt ein C₁- bis C₄-Alkenylenrest oder ein monocyclischer Rest mit 4 bis 10, insbesondere 5 bis 8 Kohlenstoffatomen;
- R⁶ =: ein q-fach substituierter, gerader, verzweigter oder cyclischer organischer Rest mit 1 bis 4 Kohlenstoffatomen, besondes bevorzugt ein C₁- bis C₃-Alkylenrest;
- p =: 1 oder 2, insbesondere 1;
- q =: 1 oder 2;
- Y =: N oder -(C=O)-NH-;
- Z =: -(C=O)-O-; und/oder
- R⁵ =: ein Wasserstoffatom oder eine Methylgruppe.

Konkrete Beispiele besonders bevorzugter Silane gemäß Formel (I) sind:

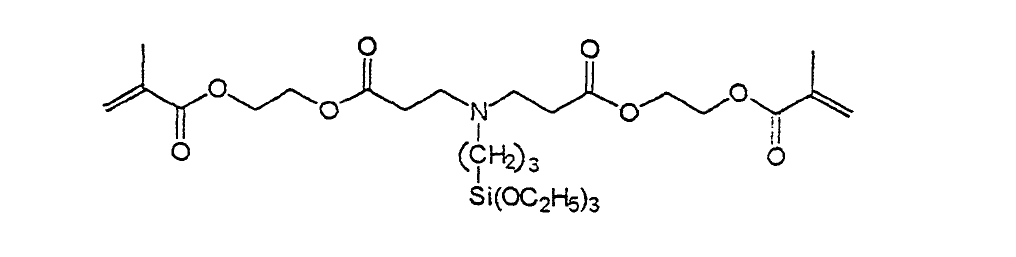

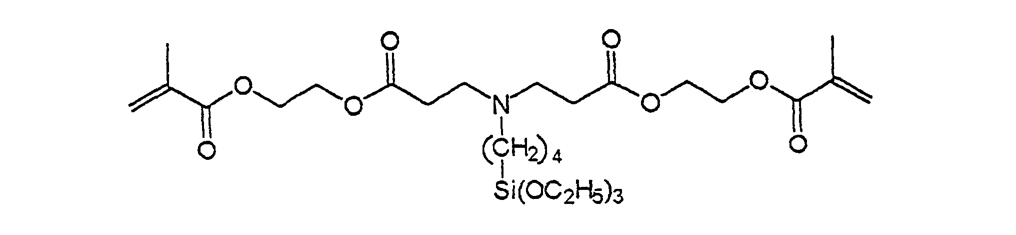

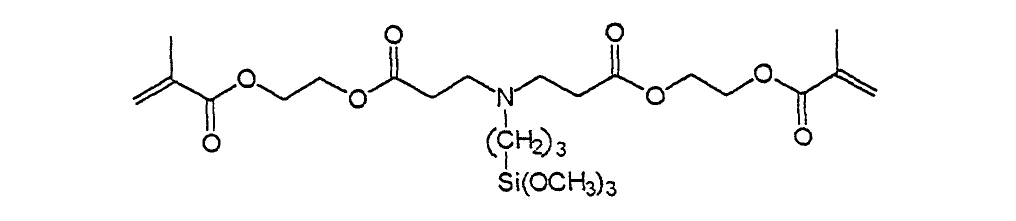

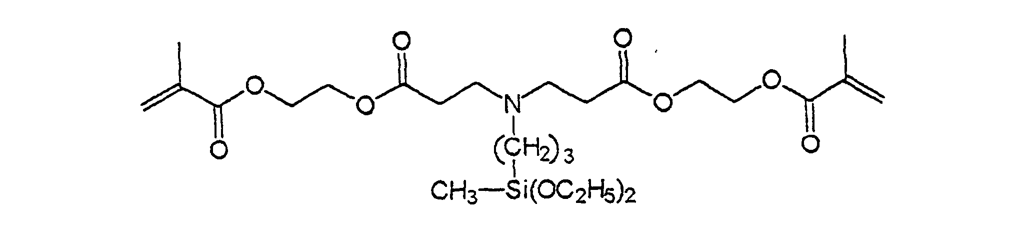

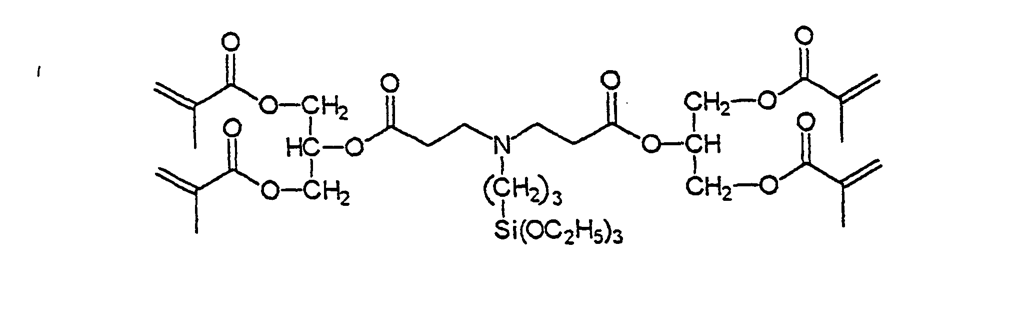

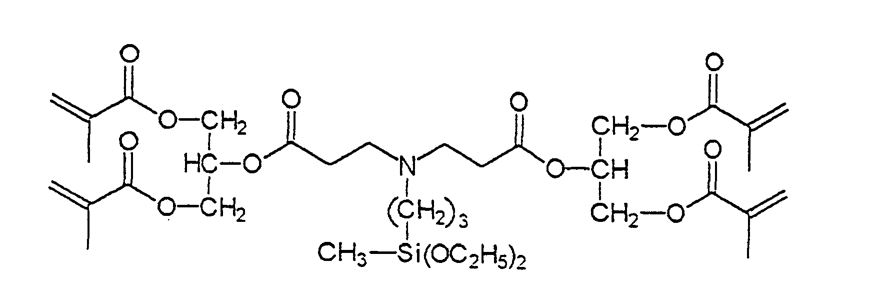

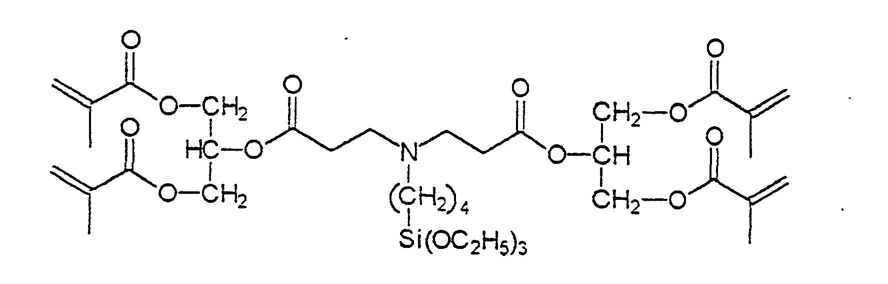

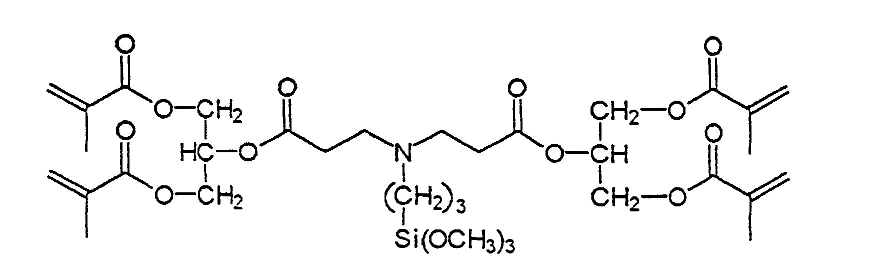

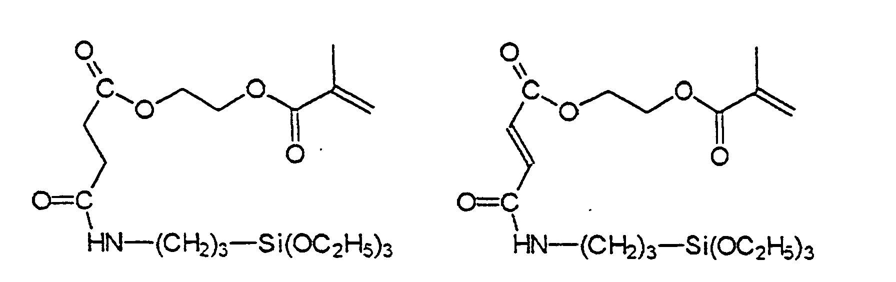

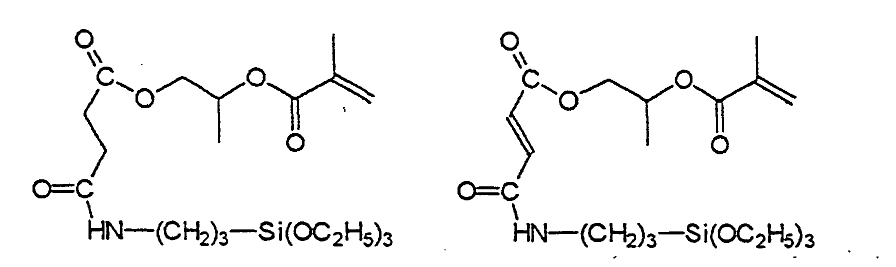

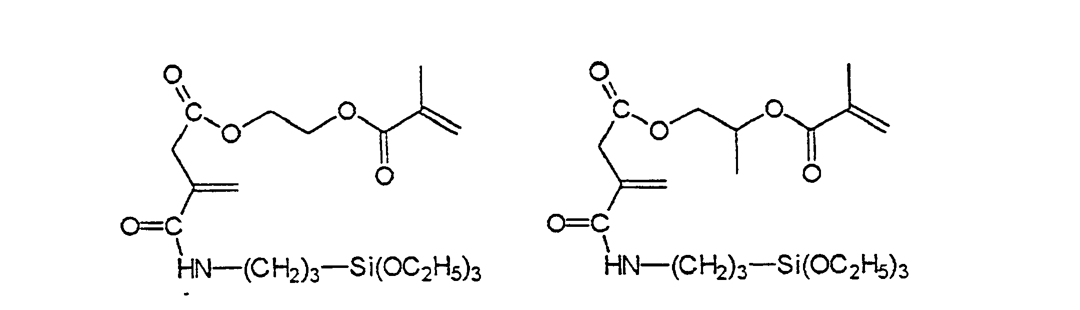

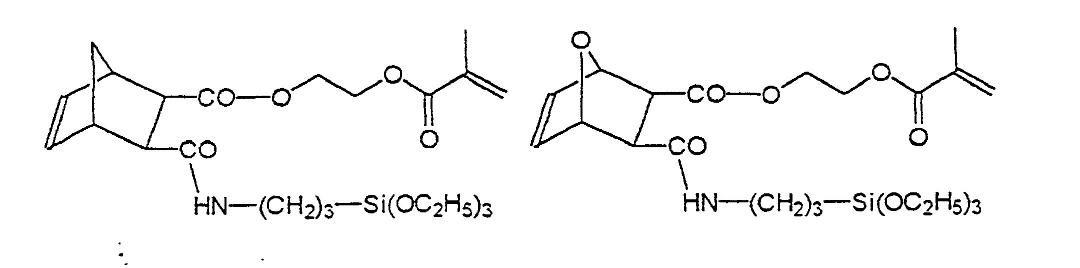

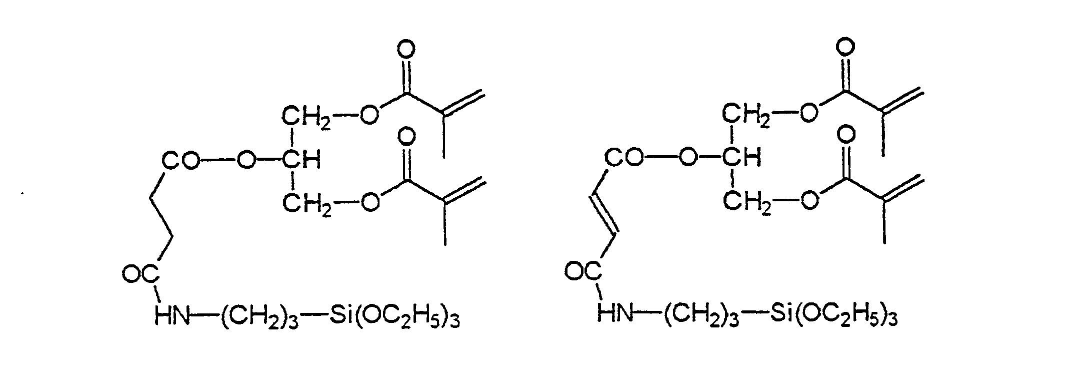

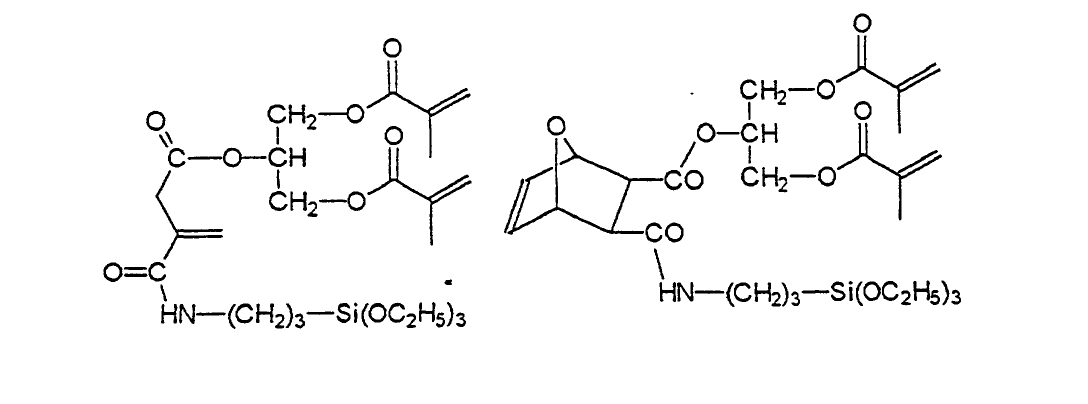

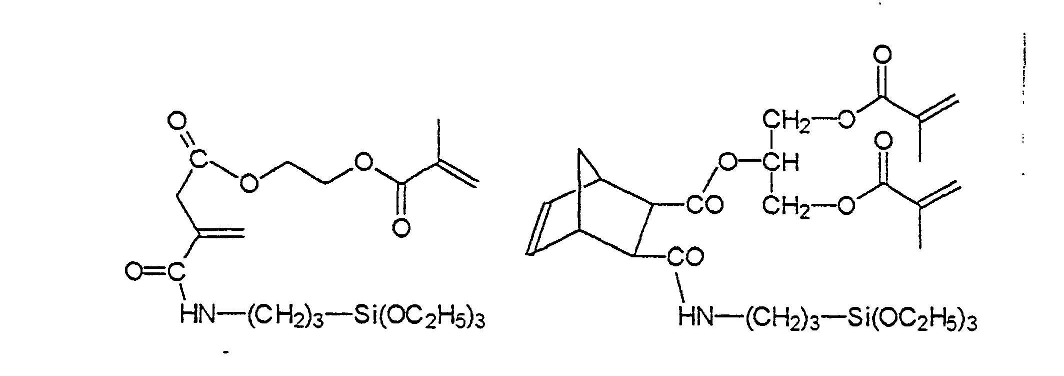

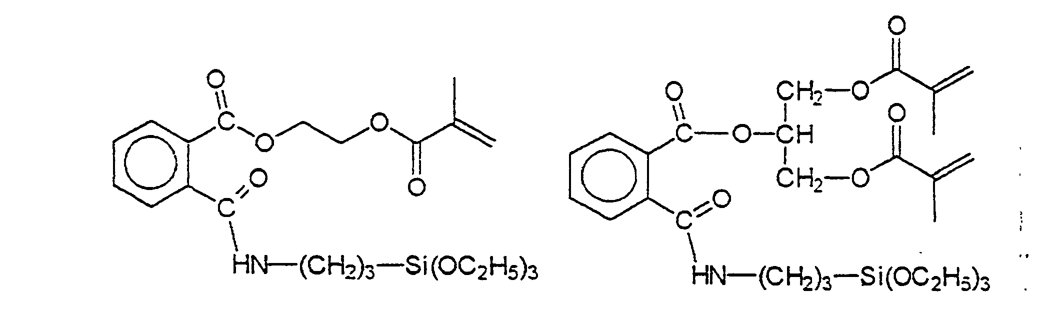

Die Silane der Formel (I) sind über an sich bekannte Additions- und Kondensationsreaktionen zugänglich, wobei durch die geeignete Auswahl der Edukte die Anzahl der hydrolysierbaren Gruppen, der polymerisationsfähigen Gruppen und weiterer funktioneller Gruppen variiert werden kann.

Silane, in denen Y die Bedeutung -NR⁴- oder N hat, sind beispielsweise durch Addition einer Aminosilanverbindung an eine m-fach ungesättigte Gruppe R³ zugänglich:

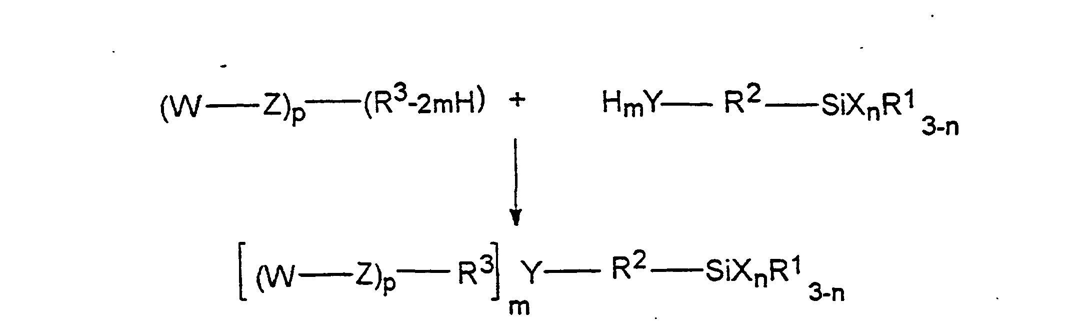

So wird z.B. durch Umsetzung von 3-Aminopropyltriethoxysilan mit 2-Acryloyloxyethylmethacrylat Bis[2-(2-methacryloyloxyethoxycarbonyl)-ethyl]-(3-triethoxysilylpropyl)amin erhalten:

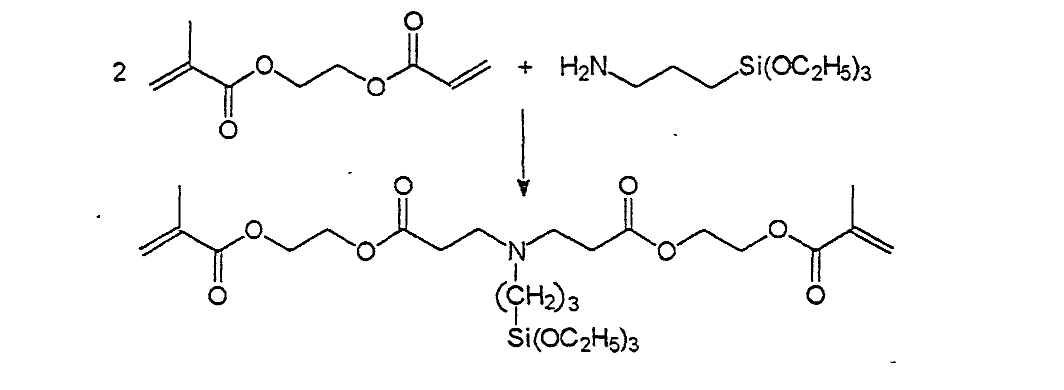

Silane in denen Y gleich -(C=O)-NH- ist, sind beispielsweise durch Reaktion eines Isocyanatosilans mit einer Carbonsäure, die p polymerisationsfähige Reste W enthält, zugänglich:

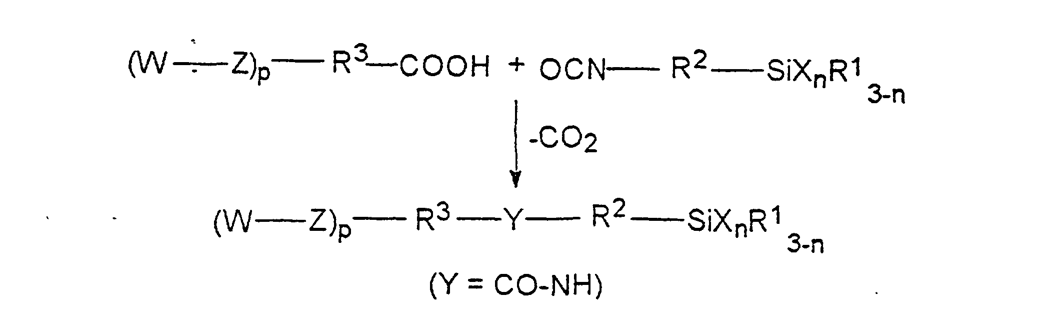

Die Reaktion von 3-Isocyanatopropyltriethoxysilan mit 2-Methacryloyloxyethylhydrogensuccinat ergibt z.B. 2-Methacryloxyethyl-3-[(3-triethoxysilyl)propylaminocarbonyl]propionat:

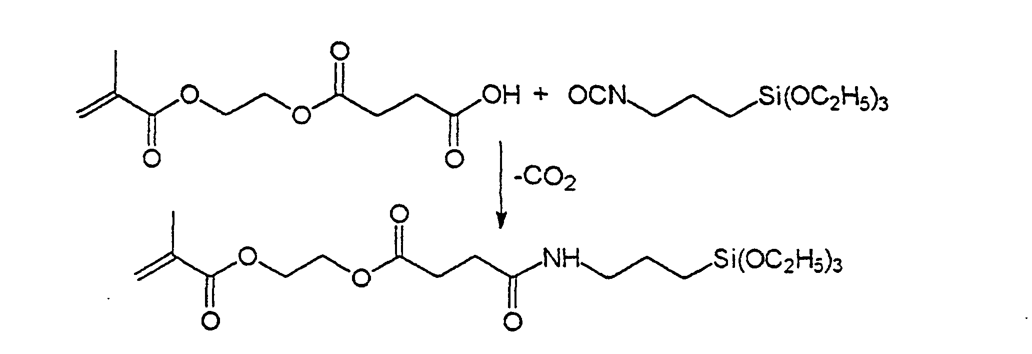

Geeignete Carbonsäuremethacrylate können durch Umsetzung von Di- oder Tetracarbonsäuremono- oder -dianhydriden mit passenden OHfunktionalisierten polymerisationsfähigen Verbindungen, wie zum Beispiel 2-Hydroxyethylmethacrylat oder Glycerindimethacrylat, erhalten werden.

Zur Synthese von Silanen in denen Y gleich -(C=O)-NH- ist, lassen sich darüber hinaus auch die in der Peptidchemie bekannten Synthesemethoden, wie z.B. die DCC-Methode oder die Methode der gemischten Anhydride, zur Umsetzung von Carbonsäuren mit aminogruppenhaltigen Verbindungen anwenden, beispielsweise die Umsetzung eines Aminosilans mit einer Carbonsäure, die p polymerisationsfähige Reste W enthält:

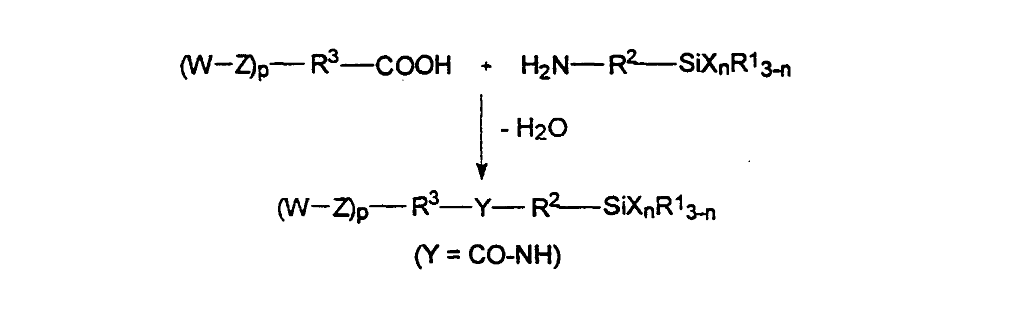

So ergibt die Reaktion von 3-Aminopropyltriethoxysilan mit 2-Methacryloyloxyethylhydrogensuccinat ebenfalls 2-Methacryloxyethyl-3-[(3-triethoxysilyl)propylaminocarbonyl]propionat:

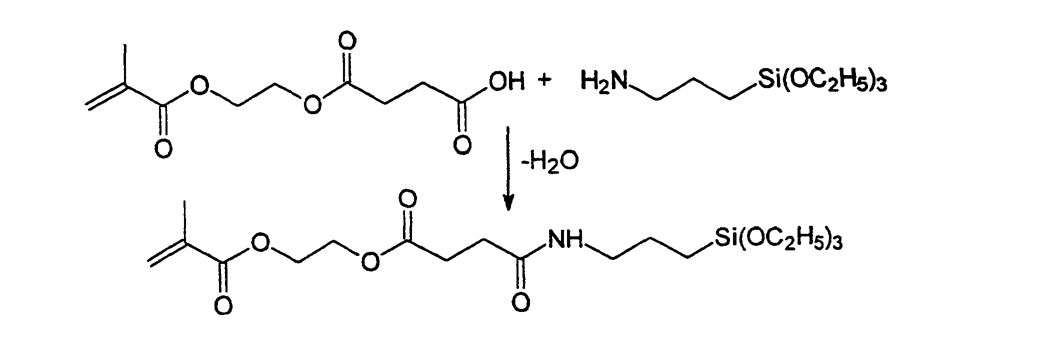

Die Silane (I) sind stabile Verbindungen und können entweder allein oder zusammen mit anderen hydrolytisch kondensierbaren Verbindungen des Siliciums, Aluminiums, Zirkoniums, Titans, Bors, Zinns, Vanadiums und/oder Phosphors zu den Polysiloxanen verarbeitet werden. Diese zusätzlichen Verbindungen können entweder als solche oder bereits in vorkondensierter Form eingesetzt werden.

Bevorzugte weitere hydrolytisch kondensierbare Verbindungen des Siliciums sind Silane der allgemeinen Formel (II)

R⁷ ₖ(Z'R⁸)ₘSiX' ₄₋₍ₖ₊ₘ₎ Formel (II)

in der
- R⁷: für eine C₁- bis C₈-Alkyl-, C₂- bis C₁₂-Alkenyl- oder C₆- bis C₁₄-Arylgruppe steht;
- R⁸: für eine C₁- bis C₈-Alkylen-, C₂- bis C₁₂-Alkenylen- oder C₆-bis C₁₄-Arylengruppe steht;
- X': für ein Wasserstoff- oder Halogenatom oder eine C₁- bis C₈-Alkoxygruppe steht;
- Z': für eine Glycidyl-, Acryl-, Methacryl-, Vinyl-, Allyl- oder Vinylethergruppe steht;
- k: gleich 0, 1, 2 oder 3 ist;
- m: gleich 0, 1, 2 oder 3 ist; und
- k+m: gleich 0, 1, 2 oder 3 ist.

Unabhängig voneinander wählbare bevorzugte Definitionen für die einzelnen Variablen sind:
- R⁷ =: eine C₁- bis C₃-Alkyl-, C₂- bis C₅-Alkenyl- oder eine Phenylgruppe;
- R⁸ =: eine C₁- bis C₅-Alkylen-, C₂- bis C₅-Alkenylen- oder eine Phenylengruppe;
- X' =: ein Halogenatom, eine Methoxy- oder Ethoxygruppe;
- Z' =: eine Acryl- oder Methacrylgruppe;
- k =: 0 oder 1;
- m =: 0 oder 1;
- k+m =: 0, 1 oder 2.

Derartige Silane sind beispielsweise in der DE 34 07 087 A1 beschrieben. Besonders bevorzugte Silane der Formel (II) sind:
CH₃-SiCl₃, CH₃-Si(OC₂H₅)₃, C₂H₅-SiCl₃, C₂H₅-Si(OC₂H₅)₃, CH₂=CH-Si(OC₂H₅)₃, CH₂=CH-Si(OCH₃)₃, CH₂=CH-Si(OC₂H₄OCH₃)₃, (CH₃)₂SiCl₂, (CH₃)₂Si(OC₂H₅)₂, (C₂H₅)₃Si-Cl, (C₂H₅)₂Si-(OC₂H₅)₂, (CH₃)₃Si-Cl, (CH₃O)₃Si-C₃H₆NH₂, (CH₃O)₃Si-C₃H₆SH, (CH₃O)₃Si-C₃H₆NH₂,

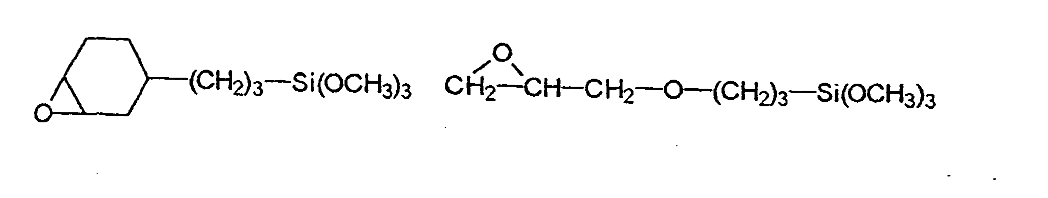

Silane der allgemeinen Formel (II) bzw. davon abgeleitete vorkondensierte Produkte werden vorzugsweise in einer Menge von 0 bis 90 mol-%, besonders bevorzugt 1 bis 60 mol-% und ganz besonders bevorzugt 1 bis 40 mol-% bezogen auf die Gesamtmasse an Silanen der Formeln (I) und (II) oder davon abgeleiteten vorkondensierten Produkten eingesetzt.

Bevorzugte Zirkonium-, Titanverbindungen sind solche gemäß Formel (III)

MeX" _{y}R⁹ _{z} Formel (III)

in der
- Me: für Zr oder Ti steht;
- R⁹: für ein Wasserstoffatom, eine substituierte oder unsubstituierte C₁- bis C₁₂-Alkyl-, C₁- bis C₁₅-Alkylaryl- oder C₆₋- bis C₁₄-Arylgruppe steht;
- X": für ein Halogenatom, eine Hydroxyl- oder C₁-bis C₈-Alkoxygruppe steht;
- Y: gleich 1 bis 4 ist;
- Z: gleich 0 bis 3 ist.

Unabhängig voneinander wählbare bevorzugte Definitionen für die einzelnen Variablen sind:
- R⁹ =: eine C₁- bis C₅-Alkyl- oder eine Phenylgruppe;
- X" =: ein Halogenatom, eine Methoxy-, Ethoxy- oder Propoxygruppe;
- Y =: 4;
- Z =: 0 oder 1, insbesondere 0.

Besonders bevorzugte Zirkonium- und Titanverbindungen sind ZrCl₄, Zr(OC₂H₅)₄, Zr(OC₃H₇)₄, Zr(OC₄H₉)₄, ZrOCl₂, TiCl₄, Ti(OC₂H₅)₄, Ti(OC₃H₇)₄ und Ti(OC₄H₉)₄.

Die Zirkonium- und Titanverbindungen der allgemeinen Formel (III) bzw. davon abgeleitete vorkondensierte Produkte werden vorzugsweise in einer Menge von 0 bis 70 mol-%, besonders bevorzugt 0 bis 50 mol-% oder 0 bis 30 mol-% und ganz besonders bevorzugt 0 bis 20 mol-% bezogen auf die Gesamtmasse an Verbindungen der Formeln (I) und (III) oder davon abgeleiteten vorkondensierten Produkten eingesetzt.

Bevorzugte Aluminiumverbindungen sind solche gemäß der Formel (IV)

AlR¹⁰ ₃ Formel (IV)

in der
- R¹⁰: für ein Halogenatom, eine Hydroxyl- oder C₁- bis C₈-Alkoxygruppe, vorzugsweise für ein Halogenatom oder eine C₁- bis C₅-Alkoxygruppe steht.

Besonders bevorzugte Aluminiumverbindungen sind Al(OCH₃)₃, Al(OC₂H₅)₃, Al(OC₃H₇)₃, Al(OC₄H₉)₃ und AlCl₃.

Die Aluminiumverbindungen der allgemeinen Formel (IV) bzw. davon abgeleitete vorkondensierte Produkte werden vorzugsweise in einer Menge von 0 bis 70 mol-%, besonders bevorzugt 0 bis 30 mol-% und ganz besonders bevorzugt 0 bis 20 mol-% bezogen auf die Gesamtmasse an Verbindungen der Formeln (I) und (IV) oder davon abgeleiteten vorkondensierten Produkten eingesetzt.

Außerdem können komplexierte Verbindungen des Zirkoniums, Titans und Aluminiums eingesetzt werden, wobei als Komplexbildner Säuren und β-Dicarbonylverbindungen bevorzugt sind. Bevorzugte Säuren sind Acryl- und Methacrylsäure oder andere Methacrylatcarbonsäuren, wie z.B. 2-Methacryloyloxyethylhydrogensuccinat oder die 1:1-Addukte aus Glycerindimethacrylat und Carbonsäureanhydriden, wie z.B. Bernsteinsäure- oder Phthalsäureanhydrid. Bevorzugte β-Carbonylverbindungen sind Acetylaceton, Acetessigsäureethylester und insbesondere 2-Acetoacetoxyethylmethacrylat. Diese Komplexbildner werden bevorzugt mit Alkoxyderivaten des Zirkoniums, Titans oder Aluminiums im Molverhältnis von 1:1 umgesetzt.

Darüber hinaus eignen sich Bortrihalogenide, Zinntetrahalogenide, Zinntetraalkoxide und/oder Vanadylverbindungen zur Cokondensation mit den Silanen gemäß Formel (I).

Bei der Verwendung zusätzlicher hydrolytisch kondensierbarer Verbindungen beträgt der Anteil von Silanen gemäß Formel (I) an den Polysiloxanen vorzugsweise 10 bis 99 mol-%, besonders bevorzugt 40 bis 99 mol-%, bezogen jeweils auf die monomeren Ausgangsverbindungen. Der Anteil an Silanen (I) und (II) zusammen beträgt vorzugsweise mindestens 20 mol-%, besonders bevorzugt mindestens 80 mol-%, ebenfalls bezogen auf die monomeren Ausgangsverbindungen.

Die Herstellung der Polysiloxane erfolgt durch hydrolytische Kondensation der oben aufgeführten Verbindungen. Im Fall der Silane der allgemeinen Formeln (I) und (II) werden hierbei zunächst die hydrolysierbaren Gruppen X abgespalten, wobei Silanole, Silandiole und Silantriole erhalten werden, die unter Wasserabspaltung zu Polysiloxanen mit einem anorganischen Netzwerk aus Si-O-Si-Einheiten kondensieren.

Die hydrolytische Kondensation der Silane erfolgt im allgemeinen indem die zu hydrolisierende Siliciumverbindung entweder direkt oder gelöst in einem geeigneten Lösungsmittel, bei Raumtemperatur oder unter leichter Kühlung mindestens mit der zur vollständigen Hydrolyse stöchiometrisch erforderlichen Wassermenge versetzt und die resultierende Mischung für eine oder mehrere Stunden gerührt wird. Als Lösungsmittel eignen sich insbesondere aliphatische Alkohole wie zum Beispiel Ethanol oder Isopropanol, Dialkylketone, wie Aceton oder Methylisobutylketon, Ether, wie zum Beispiel Diethylether oder Tetrahydrofuran (THF), Ester, wie zum Beispiel Ethyl- oder Butylacetat, und Gemische davon.

Die Hydrolyse und Kondensation der Ausgangsmischung erfolgt vorzugsweise in Gegenwart eines Kondensationskatalysators, wobei Protonen oder Hydroxylionen abspaltende Verbindungen, wie organische oder anorganische Säuren oder Basen, sowie Fluoridionen abgebende Verbindungen, wie Ammoniumfluorid oder Natriumfluorid, bevorzugt sind. Besonders bevorzugt sind flüchtige Säuren oder Basen, insbesondere Salzsäure oder Ammoniak. Es hat sich bewährt, bei der Hydrolyse und Kondensation Verfahrensweisen der Sol-Gel-Technologie zu übernehmen, wie sie zum Beispiel in C.J. Brinker et al., "Sol-Gel-Science", Academic Press, Boston, 1990, beschrieben sind.

Wird die hydrolytische Kondensation in Gegenwart von Zirkonium-, Titan- oder Aluminiumverbindungen durchgeführt, erfolgt die Wasserzugabe vorzugsweise stufenweise wobei die Temperatur vorzugsweise im Bereich von etwa 0 bis 30 °C gehalten wird. Es ist häufig vorteilhaft, daß Wasser in Form wasserhaltiger Lösungsmittel, wie zum Beispiel wäßrigem Ethanol, zuzugeben oder in situ zu erzeugen, beispielsweise durch chemische Reaktionen wie Veresterungen.

Die erhaltenen Polysiloxane können direkt oder nach teilweiser oder vollständiger Entfernung des Lösungsmittels eingesetzt werden. Häufig ist es vorteilhaft, das zur hydrolytischen Kondensation eingesetzte Lösungsmittel durch ein anderes Lösungsmittel zu ersetzen. Die Silane (I) und erst recht die Polysiloxane weisen aufgrund ihres hohen Molekulargewichts nur eine geringe Flüchtigkeit auf und lassen sich daher weitgehend unbedenklich verarbeiten. Im Hinblick auf die mechanischen Eigenschaften der Polysiloxane ist es vorteilhaft, die hydrolytische Kondensation bis zu einem Kondensationsgrad von 65 bis 95 mol-% zu führen, wobei sich der Kondensationsgrad durch ²⁹Si-NMR bestimmen läßt.

Die vollständige Aushärtung der Polysiloxane erfolgt durch Zugabe geeigneter Initiatoren und ggf. weiterer polymerisationsfähiger Komponenten durch thermische, photochemische oder redoxinduzierte Polymerisation. Dabei können bei Vorhandensein unterschiedlicher polymerisationsfähiger Gruppen, z.B. von (Meth)acryl- und Epoxidgruppen, auch mehrere Härtungsmechanismen, z.B. radikalische und kationische Polymerisation, gleichzeitig oder in aufeinander folgenden Stufen zur Anwendung kommen.

Zur Initiierung der radikalischen Polymerisation werden vorzugsweise thermische und/oder Photoinitiatoren verwendet.

Bevorzugte Initiatoren für die thermische Härtung sind Peroxide, wie beispielsweise Dibenzoylperoxid, Dilaurylperoxid, tert.-Butylperoctoat und tert.-Butylperbenzoat sowie Azobisisobutyroethylester, Benzpinakol und 2,2-Dimethylbenzpinakol.

Bevorzugte Photoinitiatoren sind Benzophenon und Benzoin sowie deren Derivate, α-Diketone und deren Derivate, wie beispielsweise 9,10-Phenanthrenchinon, Diacetyl und 4,4-Dichlorbenzil. Besonders bevorzugte Photoinitiatoren sind Champherchinon und 2,2-Methoxy-2-phenyl-acetophenon und insbesondere Kombinationen von α-Diketonen mit Aminen als Reduktionsmittel, wie zum Beispiel N-Cyanoethyl-N-methylanilin, 4-(N,N-Dimethylamino)-benzoesäureester, N,N-Dimethylaminoethylmethacrylat, N,N-Dimethyl-sym.-xylidin oder Triethanolamin. Darüber hinaus sind Acylphosphine, wie zum Beispiel 2,4,6-Trimethylbenzoyldiphenyl- oder Bis-(2,6-dichlorbenzoyl)-4-N-propylphenylphosphinoxid, als Photoinitiatoren geeignet.

Für die duale Aushärtung von radikalisch und kationisch polymerisierbaren Systemen eignen sich besonders Diaryliodonium- oder Triarylsulfoniumsalze, wie zum Beispiel Triphenylsulfoniumhexafluorophosphat und -hexafluoroantimonat.

Als Initiatoren für eine Polymerisation bei Raumtemperatur werden Redox-Initiatorkombinationen, wie zum Beispiel Kombinationen von Benzoyl- oder Laurylperoxid mit N,N-Dimethyl-sym.-xylidin oder N,N-Dimethyl-p-toluidin, verwendet.

Die Polymerisation von Polysiloxanen mit 2 oder mehr (Meth)acrylatresten ergibt dreidimensionale, organische Netzwerke, wobei sich die mechanischen Eigenschaften, wie zum Beispiel Festigkeit und Flexibilität, sowie die physikalisch-chemischen Eigenschaften der gehärteten Materialien, wie zum Beispiel das Haftungsvermögen, Wasseraufnahme und Brechungszahl, über den Abstand zwischen den Si-Atomen und den polymerisationsfähigen (Meth)acrylatresten, d.h. über die Länge der Spacer-Gruppe -R²-Y-R³-Z-R⁶-, sowie über die Anwesenheit weiterer funktioneller Gruppen variieren und den Anforderungen des jeweiligen Anwendungsfalles optimal anpassen lassen. Dabei ergibt die Verwendung aliphatischer Gruppen als Spacer relativ flexible und die Verwendung aromatischer Gruppen relativ steife Produkte.

Durch die Anzahl der polymerisationsfähigen (Meth)acrylatgruppen läßt sich die Vernetzungsdichte der ausgehärteten Materialien einstellen, was eine weitere Beeinflussung der Eigenschaften und Einsatzmöglichkeiten der Polysiloxane erlaubt.

Enthalten die monomeren Silane darüber hinaus ionisch vernetzbare Gruppen, wie zum Beispiel Epoxid- oder Oxethangruppen, kann durch deren gleichzeitige oder anschließende ionische Polymerisation eine weitere Erhöhung der Vernetzungsdichte erreicht werden.

Die Polysiloxane können in Mischung mit geeigneten ionisch und/oder radikalisch polymerisierbaren mono- oder multifunktionellen Monomeren eingesetzt werden. Bevorzugte Monomere sind Mono(meth)acrylate, wie Methyl-, Ethyl-, Butyl-, Benzyl-, Furfuryl- oder Phenyl(meth)acrylat, mehrfunktionelle Acrylate und Methacrylate wie zum Beispiel Bisphenol-(A)-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Hexamethylendiisocyanat), Di-, Tri- und Tetraethylenglykoldi(meth)acrylat, Decandioldi(meth)acrylat, Trimethylolpropantri(meth)acrylat, Pentaerythrittetra(meth)acrylat und Butandioldi(meth)acrylat, 1,10-Decandioldi(meth)acrylat oder 1,12-Dodecandioldi(meth)acrylat.

Die polymerisierbaren Monomere werden vorzugsweise in einer Menge von 1 bis 80 Gew.-%, besonders bevorzugt 5 bis 50 Gew.-% und ganz besonders bevorzugt 5 bis 30 Gew.% bezogen auf die Gesamtmasse an polymerisierbarem Monomer und Silan der Formeln (I) oder davon abgeleiteten vorkondensierten Produkten eingesetzt.

Die Mischungen können darüber hinaus weitere Additive wie Färbemittel (Pigmente und Farbstoffe), Stabilisatoren, Aromastoffe, mikrobiozide Wirkstoffe, Weichmacher und/oder UV-Absorber enthalten.

Weiterhin können die Zusammensetzungen zur Verbesserung der mechanischen Eigenschaften mit organischen oder anorganischen Partikeln oder Fasern gefüllt werden. Bevorzugte anorganische partikuläre Füllstoffe sind amorphe kugelförmige Materialien auf der Basis von Mischoxiden aus SiO₂, ZrO₂ und/oder TiO₂ (DE 40 29 230 A1), mikrofeine Füllstoffe, wie pyrogene Kieselsäure oder Fällungskieselsäure sowie Makro- (Partikelgröße von 5 µm bis 200 µm) oder Minifüllstoffe (Partikelgröße von 0,5 bis 5 µm), wie Quarz-, Glaskeramik- oder Glaspulver mit einer durchschnittlichen Teilchengröße von 0,5 µm bis 5 µm sowie röntgenopake Füllstoffe, wie Ytterbiumtrifluorid. Darüber hinaus können auch Glasfasern, Polyamid- oder Kohlenstoffasern als Füllstoffe eingesetzt werden.

Die Zusammensetzungen eignen sich besonders als Dentalmaterialien, wie Adhäsive, Beschichtungsmaterialien, dentale Zemente und Füllungsmaterialien.

Die erfindungsgemäßen Dentalmaterialien enthlaten vorzugsweise
(a) 5 bis 99,9 Gew.-%, vorzugsweise 5 bis 90 Gew.-%, besonders bevorzugt 10 bis 70 Gew.-% Polysiloxan; und
(b) 0,1 bis 5,0 Gew.-%, vorzugsweise 0,2 bis 2,0 Gew.-% Polymerisationsinitiator; und vorzugsweise
(c) 1,0 bis 80 Gew.-%, vorzugsweise 5,0 bis 50 Gew.-% ionisch und/oder radikalisch polymerisierbares Monomer; und vorzugsweise
(d) 1,0 bis 90 Gew.-%, vorzugsweise 2,0 bis 80 Gew.-% Füllstoffe.

Die Angaben beziehen sich jeweils auf die Gesamtmasse des Dentalmaterials.

Die Erfindung wird im folgenden anhand von Ausführungsbeispielen näher erläutert.

### Beispiel 1

### Synthese von Bis[2-(2-methacryloyloxyethoxycarbonyl)-ethyl]-(3-triethoxysilylpropyl)amin

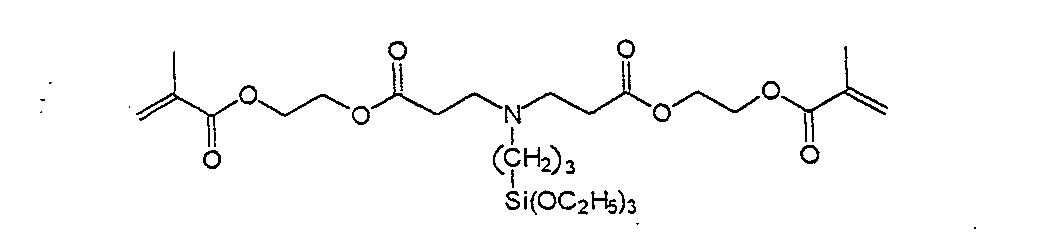

43 g (0,25 mol) 3-Aminopropyltriethoxysilan in 40 ml wasserfreiem Acetonitril werden unter Eiskühlung zu 92 g (0,5 mol) 2-Acryloyloxyethylmethacrylat in 85 ml Acetonitril getropft. Nach 48 h Rühren bei Raumtemperatur hat sich das Aminosilan vollständig umgesetzt. Das Lösungsmittel wird unter Einleiten von Luft bei vermindertem Druck am Rotationsverdampfer bei maximal 46 °C abgedampft. Es werden 132,2 g (98 % Ausbeute) einer leichtgefärbten klaren öligen Flüssigkeit erhalten.

¹H-NMR (400 MHz, CDCl₃): δ = 0,58 (t, 2H, SiCH₂), 1,22 (t, 9H, CH₃), 1,52 (m, 2H, CH₂), 1,95 (s, 3H, CH₃=C), 2,45 (m, 6H, NCH₂), 2,78 (t, 4H, O=C-CH₂), 3,82 (q, 6H, OCH₂CH₃), 4,34 (s, 8H, OCH₂CH₂O), 5,59 und 6,16 (2s, 4H, =CH₂) ppm.

IR (Film): 2974 (s), 1724 (s), 1637 (w), 1320 (m), 1296 (m), 1163 (s) und 1078 (m) cm⁻¹.

### Beispiel 2

### Synthese des Adduktes von 3-Aminopropylsilan an 2(1)-Acryloyloxy-1(2),3-dimethacryloyloxypropan

### 1. Stufe: 2(1)-Acryloyloxy-1(2),3-di(methacryloyloxy)propan

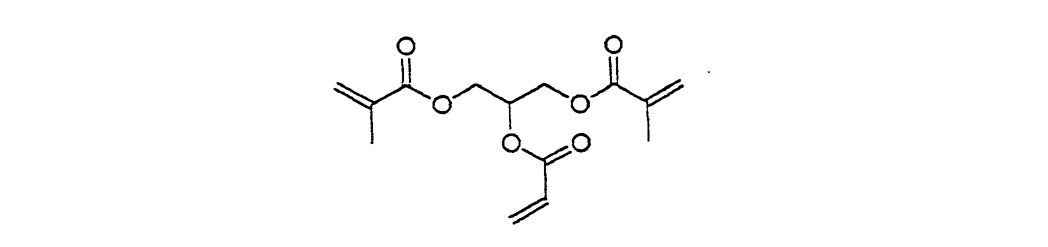

64 g (0,7 mol) Acrylsäurechlorid in 290 ml Ether werden zu einer eisgekühlten Lösung von 135 (0,6 mol) Glycerindimethacrylat (GDMA) und 85 g (0,7 mol) Collidin in 290 ml Ether getropft. Nach 16 h Rühren bei Raumtemperatur wird der gebildete Niederschlag an Collidiniumhydrochlorid abfiltriert und die Etherlösung 2mal mit je 100 ml 1 N HCl, 2mal mit je 100 ml 10%iger Natriumhydrogencarbonatlösung und 3mal mit je 100 ml Wasser gewaschen. Die Etherphase wird über wasserfreiem Na₂SO₄ getrocknet, mit 20 mg Hydrochinonmonomethylether (MEHQ) stabilisiert, und das Lösungsmittel unter Lufteinleiten am Rotationsverdampfer bei vermindertem Druck abdestilliert. Es werden 79 g (98 % Ausbeute) einer dunkelgelben, klaren Flüssigkeit erhalten.

¹H-NMR (400 MHz, CDCl₃): δ = 1,94 (s, 6H, CH₃), 4,20-4,44 und 5,48 (m, 5H, CHO, CH₂O) und 5,60-6,78 (m, 7H, =CH₂, CH=CH₂) ppm.

Aus dem detaillierten Vergleich der Integralverhältnisse geht hervor, daß die Umsetzung vollständig ist und das Produkt 13 % GDMA enthält.

IR (Film): 2960 (m), 1728 (s), 1638 (s), 1407 (s), 1294 (s) und 1163 (s) cm⁻¹.

### 2. Stufe: Umsetzung von 3-Aminopropylsilan mit 2(1)-Acryloyloxy-1(2),3-di(methacryloyloxy)propan

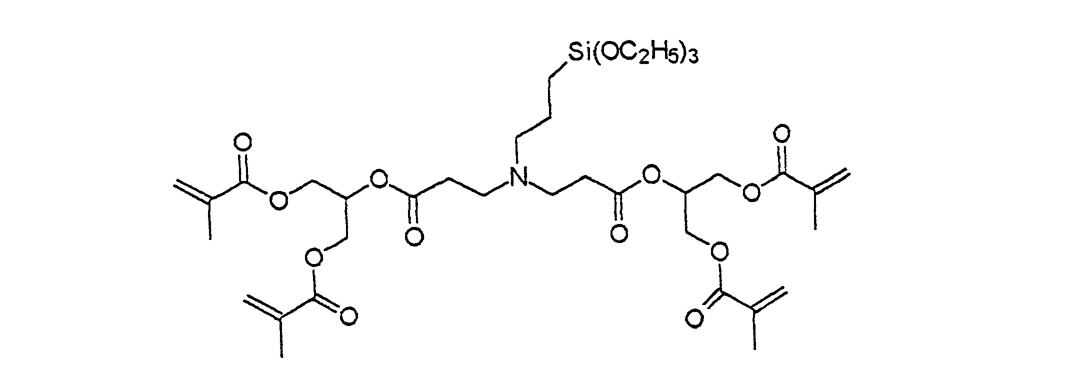

Eine Lösung von 63,3 g (224 mmol) 2(1)-Acryloyloxy-1(2),3-dimethacryloyloxypropan, 24,8 g (111 mmol) 3-Aminopropyltriethoxysilan, 50 mg MEHQ in 200 ml absolutem Methanol wird 6 d unter Argon bei 40 °C gerührt. Unter Einleiten von trockener Luft wird das Methanol bei 40 °C und reduziertem Druck am Rotationsverdampfer entfernt. Es werden 79 g (89 % Ausbeute) einer dunkelgelben, klaren Flüssigkeit erhalten.

¹H-NMR (400 MHz, CDCl₃): δ = 0,59 (t, 2H, SiCH₂), 1,25 (q, CH₃), 1,50-1,52 (m, 2H, CH₂), 1,96 (s, 12H, CH₃), 2,42-2,44 (m, 6H, NCH₂), 2,76 (t, 4H, O=C-CH₂), 3,56 (s, OCH₃), 3,80 (t, OCH₂), 4,19-4,40 und 5,18 (m, 10H, CHO und CH₂O) und 5,60 und 6,15 (2s, 8H, =CH₂) ppm.

Aus dem ¹H-NMR-Spektrum geht hervor, daß an der Triethoxysilylgruppe während der Umsetzung teilweise eine Umesterung mit dem Lösungsmittel Methanol erfolgte.

IR (Film): 3504 (w), 2954 (m), 1724 (s), 1637 (m), 1453 (m), 1296 (s) und 1162 (s) cm⁻¹.

### Beispiel 3

### Synthese von 2-Methacryloyloxyethyl-3-[(3-triethoxysilyl)-propylaminocarbonyl]propionat

### 1. Stufe: 2-Methacryloyloxyethylhydrogensuccinat

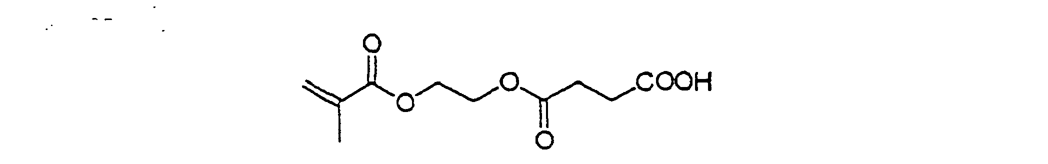

40 g (0,4 mol) Bernsteinsäureanhydrid, 52 g (0,4 mol) HEMA und 80 mg MEHQ in 200 ml Dioxan werden mit 2 Tropfen konz. Schwefelsäure versetzt und 5 h auf 80 °C erwärmt. Das Dioxan wird am Rotationsverdampfer bei angelegtem Ölpumpenvakuum (1-5 mbar) unter Lufteinleiten weitgehend abdestilliert. Das Produkt wird dann in 100 ml Methylenchlorid aufgenommen und 3mal mit je 100 ml Wasser gewaschen und über wasserfreiem Natriumsulfat getrocknet. Nach erneutem Stabilisieren mit 40 mg MEHQ wird das Lösungsmittel unter vermindertem Druck und Lufteinleiten entfernt. Es werden 80 g (87 % Ausbeute) einer gelbgefärbten Flüssigkeit erhalten.

¹H-NMR (400 MHz, CDCl₃): δ = 1,93 (s, 3H, CH₃), 2,53-2,64 (m, 4H, CH₂CH₂COOH), 4,31 (s, 4H, OCH₂CH₂O), 5,61 und 6,11 (2s, 2H, =CH₂) und 10,70 (br, 1H, COOH) ppm.

IR (Film): 3400-2400 (br), 2960 (m), 1722 (s), 1698 (s), 1636 (m), 1406 (m) und 1147 (s) cm⁻¹.

### 2. Stufe: 2-Methacryloyloxyethyl-3-[(3-triethoxysilyl)propylaminocarbonyl]-propionat

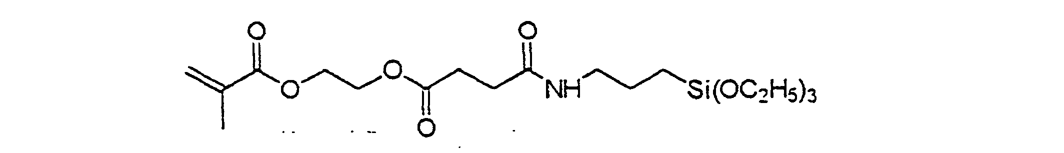

20 g (87 mmol) 2-Methacryloyloxyethylhydrogensuccinat, 21,5 g (87 mmol) 3-Isocyanatopropyltriethoxysilan und 2 Tropfen Dibutylzinndioctoat werden bei Raumtemperatur in 50 ml Methylenchlorid solange gerührt bis im IR-Spektrum kein Isocyanat mehr nachweisbar ist (ca. 3 Tage). Die Reaktionslösung wird mit 30 mg MEHQ stabilisiert und das Lösungsmittel unter Einleiten von trockener Luft am Rotationsverdampfer abdestilliert. Es werden 33,7 g (90 % Ausbeute) einer gefärbten Flüssigkeit erhalten.

¹H-NMR (400 MHz, CDCl₃): δ = 0,65 (t, 2H, CH₂Si), 1,23 (t, 9H, CH₃), 1,61-1,62 (m, 2H, CH₂), 1,95 (s, 3H, CH₃), 2,51-2,67 (m, 4H, CH₂CH₂CO₂), 3,10-3,20 (m, 2H, CH₂N), 3,81 (q, 6H, CH₂O), 4,27 (br, 1H, NH), 4,37 (s, 4H, OCH₂CH₂O) und 5,45 und 6,14 (2s, 2H, =CH₂) ppm.

IR (Film): 3346 (w), 2975 (m), 1724 (s), 1639 (w), 1297 (m) und 1159 (s) cm⁻¹.

### Beispiel 4

### Hydrolytische Kondensation von Bis(methacryloylethoxycarbonylethyl)-[3-(triethoxysilylpropyl)]amin

100 mmol des Silans aus Beispiel 1 werden in 30 ml wasserfreiem Ethanol gelöst. Die Vorhydrolyse des Silans erfolgt durch Zugabe von 300 mmol Wasser in Form einer 0,1 N NH₄F-Lösung. Nach 16 bis 20 h Rühren bei Raumtemperatur werden die flüchtigen Komponenten im Vakuum entfernt, und es entsteht ein niedrigviskoses Harz (η = 1,6 Pas), das nach Zugabe eines radikalischen Initiators als Komponente für eine lichthärtende Beschichtung oder ein lichthärtendes Dentalmaterial eingesetzt werden kann.

### Beispiel 5

### Hydrolytische Kondensation von Bis(methacryloylethoxycarbonylethyl)-[3-(triethoxysilylpropyl)]amin und anschließende Silylierung

100 mmol des Silans aus Beispiel 1 werden in 250 ml EtOH gelöst. Die Hydrolyse des Silans erfolgt durch Zugabe von 300 mmol Wasser in Form einer 0,1 N NH₄F-Lösung. Nach 16 bis 22 h Rühren bei Raumtemperatur werden die flüchtigen Komponenten im Vakuum entfernt. Das entstandene viskose Harz wird in 80 ml THF gelöst und zur Silylierung noch vorhandener Si-OH-Gruppen mit 100 mmol Collidin als Base und 100 mmol Trimethylchlorsilan (TMCS) unter Kühlung versetzt. Die Mischung wird zur Vervollständigung der Reaktion 12 bis 24 h bei Raumtemperatur gerührt, bevor der entstandene Niederschlag abfiltriert wird. Nach dem Entfernen der flüchtigen Komponenten im Vakuum erhält man ein niedrigviskoses Harz (η = 4,9 Pas (23 °C)), das nach Zugabe eines radikalischen Initiators als solches oder als Komponente für eine lichthärtende Beschichtung oder ein lichthärtendes Dentalmaterial eingesetzt werden kann.

Zur Bestimmung der mechanischen Eigenschaften wurden aus dem Harz entsprechende Prüfkörper hergestellt und durch Belichtung (6 Minuten) mit einer dentalen Strahlungsquelle Spectramat (Vivadent) ausgehärtet. Der Polymerisationsschrumpf (ΔV) wurde aus der Differenz der gaspyknometrisch bestimmten Harz- und Polymerdichte berechnet und die Biegefestigkeit (BF) bzw. der Biege-E-Modul (BEM), nach der ISO-Norm 4049 (1988) bestimmt, wobei die Prüfkörper jeweils bei 37 °C für 24 h getrocknet wurden. Die Ergebnisse sind: BF = 67 MPa, BEM = 1950 MPa, ΔV = -7,3 Vol-%. Mittels dynamisch-mechanischer Analyse wurde eine Glasübergangstemperatur des Polymerisates von T_{G} = 95 °C bestimmt.

### Beispiel 6

### Hydrolytische Kondensation des Adduktes von 3-Aminopropylsilan mit 2(1)-Acryloyloxy-1(2),3-di(methacryloyloxy)propan

100 mmol des Silans aus Beispiel 2 werden in 300 ml wasserfreiem Ethanol gelöst. Die Vorhydrolyse des Silans erfolgte durch Zugabe von 150 mmol Wasser in Form eine 0,1 N NH₄F-Lösung. Nach 16 bis 20 h Rühren bei Raumtemperatur werden die flüchtigen Komponenten im Vakuum entfernt und ein niedrigviskoses Harz (η = 2,8 Pas) erhalten, das nach Zugabe eines radikalischen Initiators als Komponente für eine lichthärtende Beschichtung oder ein lichthärtendes Dentalmaterial eingesetzt werden kann.

### Beispiel 7

### Herstellung eines Dentalzements

Auf der Basis des nach hydrolytischer Kondensation und anschließender Silylierung erhaltenen Harzes aus Beispiel 5 wird eine Dentalzement folgender Rezeptur hergestellt:

| | |
|---|---|
| Harz aus Beispiel 5: | 31,6 Gew.-% |
| UDMA: | 7,8 Gew.-% |
| OX-50 silanisiert: | 41,4 Gew.-% |
| YbF₃: | 18,7 Gew.-% |
| Photoinitiator*): | 0,5 Gew.-% |
| OX-50 = silanisierte Pyrolysekieselsäure (Fa. Degussa), Primärpartikelgröße 40 nm YbF₃ = Ytterbiumfluorid (Fa. Rhone-Poulenc) | |

| | |
|---|---|
| *) Mischung aus gleichen Anteilen an Campherchinon und N-(2-Cyanoethyl)-N-methylanilin | |

Die Komponenten werden mit einem Dreiwalzenstuhl des Typs Exakt (Fa. Exakt Apparatebau) zu einer Paste verarbeitet und dann analog zu Beispiel 5 Prüfkörper hergestellt und getestet. Die Ergebnisse sind: BF = 62 MPa, BEM = 3260 MPa und ΔV = -3,6 Vol-%.

## Patentansprüche

1. Dentalmaterial, enthaltend mindestens ein Polysiloxan auf der Basis von einem oder mehreren Silanen gemäß der Formel (I)
[(W_{q}-R⁶-Z)ₚ-R³]ₘY-R²-SiXₙR¹ ₃₋ₙ Formel (I)
in der
X für ein Halogenatom, eine Hydroxyl-, Alkoxy- und/oder Acyloxygruppe steht;
n gleich 1 bis 3 ist;
R¹ für eine Alkyl-, Alkenyl-, Aryl-, Alkylaryl-, Arylalkylgruppe steht;
R² für eine Alkylengruppe steht;
R³ für einen p-fach substituierten, geraden, verzweigten oder cyclischen, gesättigten oder ungesättigten, aromatischen oder aliphatischen organischen Rest mit 2 bis 40 Kohlenstoffatomen und ggf. 1 bis 6 Heteroatomen steht;
R⁶ für einen q-fach substituierten, geraden, verzweigten oder cyclischen organischen Rest mit 1 bis 20 Kohlenstoffatomen steht;
p gleich 1 bis 6 ist;
q gleich 1 bis 6 ist;
Y für -NR⁴-, N oder -(C=O)-NH- steht;
m gleich 2 für Y = N und gleich 1 für Y = -NR⁴- oder -(C=O)-NH- ist;
R⁴ Z für eine Alkyl- oder Arylgruppe steht; für O, S, -(C=O)-O-, -(C=O)-NH-, -O-(C=O)-NH- steht oder entfällt;
W für CH₂=CR⁵-(C=O)-O- steht; und
R⁵ für ein Wasserstoffatom oder eine Alkylgruppe steht
und ggf. einer oder mehreren weiteren hydrolytisch kondensierbaren Verbindungen des Siliciums, Aluminiums, Zirkoniums, Titans, Bors, Zinns, Vanadiums und/oder Phosphors.

2. Dentalmaterial nach Anspruch 1, **dadurch gekennzeichnet, daß**
X für eine Methoxy- und/oder Ethoxygruppe steht;
n 2 oder 3 ist;
R¹ für eine C₁- bis C₃-Alkylgruppe, insbesondere eine Methylgruppe steht;
R² für eine C₁- bis C₄-Alkylengruppe steht;
R³ für einen p-fach substituierten, geraden, verzweigten oder cyclischen, gesättigten oder ungesättigten, aromatischen oder aliphatischen organischen Rest mit 2 bis 10 Kohlenstoffatomen und ggf. einem Heteroatom, vorzugsweise einem Sauerstoffatom, insbesondere für C₁- bis C₄-Alkenylen oder einen monocyclischen Rest mit 4 bis 10 Kohlenstoffatomen steht;
R⁶ für einen q-fach substituierten, geraden, verzweigten oder cyclischen organischen Rest mit 1 bis 4 Kohlenstoffatomen, insbesondere einen C₁- bis C₃-Alkylenrest steht;
p gleich 1 oder 2, insbesondere 1 ist;
q gleich 1 oder 2 ist;
Y für N oder -(C=O)-NH- steht;
Z für -(C=O)-O-; und/oder
R⁵ für ein Wasserstoffatom oder eine Methylgruppe steht.

3. Dentalmaterial nach Anspruch 1 oder 2, **dadurch gekennzeichnet, daß** die weitere hydrolytisch kondensierbare Verbindung ein Silan gemäß der Formel (II)
R⁷ ₖ(Z'R⁸)ₘSiX' ₄₋₍ₖ₊ₘ₎ (Formel (III)
in der
R⁷ für eine C₁- bis C₈-Alkyl-, C₂- bis C₁₂-Alkenyl- oder C₆-bis C₁₄-Arylgruppe steht;
R⁸ für eine C₁- bis C₈-Alkylen-, C₂- bis C₁₂-Alkenylen- oder C₆- bis C₁₄-Arylengruppe steht;
X' für ein Wasserstoff- oder Halogenatom oder eine C₁- bis C₈-Alkoxygruppe steht;
Z' für eine Glycidyl-, Acryl-, Methacryl-, Vinyl-, allyloder Vinylethergruppe steht;
k gleich 0, 1, 2 oder 3 ist;
m gleich 0, 1, 2 oder 3 ist; und
k+m gleich 0, 1, 2 oder 3 ist;
eine Zirkonium-, Titanverbindung der Formel (III)
MeX" _{y}R⁹ _{z} Formel (III)
in der
Me für Zr oder Ti steht;
R⁹ für ein Wasserstoffatom, eine substituierte oder unsubstituierte C₁- bis C₁₂-Alkyl-, C₁- bis C₁₅-Alkylaryloder C₆₋- bis C₁₄-Arylgruppe steht;
X" für ein Halogenatom, eine Hydroxyl- oder C₁-bis C₈-Alkoxygruppe steht;
Y gleich 1 bis 4 ist; und
Z gleich 1 bis 3 ist;
eine Aluminiumverbindung gemäß der Formel IV
Al R¹⁰ ₃ Formel Formel (IV)
in der
R¹⁰ für ein Halogenatom, eine Hydroxyl- oder C₁- bis C₈-Alkoxygruppe steht;
und/oder ein Bortrihalogenid, Zinntetrahalogenid, Zinntetraalkoxid und/oder eine Vanadylverbindung ist.

4. Dentalmaterial nach Anspruch 3, **dadurch gekennzeichnet, daß**
R⁷ = eine C₁- bis C₃-Alkyl-, C₂- bis C₅-Alkenyl- oder eine Phenylgruppe ist;
R⁸ = eine C₁- bis C₅-Alkylen-, C₂- bis C₅-Alkenylen- oder eine Phenylengruppe ist;
X' = ein Halogenatom, eine Methoxy- oder Ethoxygruppe ist;
Z' = eine Acryl- oder Methacrylgruppe ist;
k = 0 oder 1 ist;
m = 0 oder 1 ist;
k+m= 0, 1 oder 2 ist;
und/oder
R⁹ = eine C₁- bis C₅-Alkyl- oder eine Phenylgruppe ist;
X'' = ein Halogenatom, eine Methoxy-, Ethoxy- oder Propoxygruppe ist;
Y = 4 ist;
Z = 0 oder 1, insbesondere 0 ist;
und/oder
R¹⁰= ein Halogenatom oder eine C₁- bis C₅-Alkoxygruppe ist.

5. Dentalmaterial nach Anspruch 3 oder 4, **dadurch gekennzeichnet, daß** es
das oder die Silane gemäß der allgemeinen Formel (II) oder davon abgeleitete vorkondensierte Produkte in einer Menge von 1 bis 90 mol-%, vorzugsweise 1 bis 60 mol-% und insbesondere 1 bis 40 mol-%; und/oder
die Zirkonium- und/oder Titanverbindung der allgemeinen Formel (III) oder davon abgeleitete vorkondensierte Produkte in einer Menge von 0 bis 70 mol-%, vorzugsweise 0 bis 50 mol-% und insbesondere 0 bis 30 mol-%; und/oder
die Aluminiumverbindung der allgemeinen Formel (IV) oder davon abgeleitete vorkondensierte Produkte in einer Menge von 0 bis 70 mol-%, vorzugsweise 0 bis 30 mol-% und insbesondere 0 bis 20 mol-% enthält,
bezogen auf die Gesamtmasse an Verbindungen der Formel (I) und/oder davon abgeleiteten vorkondensiertern Produkten und Verbindungen der Formel (II), Formel (III) bzw. (IV).

6. Dentalmaterial nach einem der Ansprüche 1 bis 5, **dadurch gekennzeichnet, daß** das Polysiloxan, bezogen auf die monomeren Ausgangsverbindungen, 10 bis 99 mol-% eines Silans gemäß der Formel (I) enthält.

7. Dentalmaterial nach einem der Ansprüche 1 bis 6, **dadurch gekennzeichnet, daß** es zusätzlich ein ionisch und/oder radikalisch polymerisierbares Monomer enthält.

8. Dentalmaterial nach Anspruch 7, **dadurch gekennzeichnet, daß** es ein Methyl-, Ethyl-, Butyl-, Benzyl-, Furfuryl- und/oder Phenyl(meth)acrylat, Bisphenol-A-di(meth)acrylat, Bis-GMA, UDMA, Di-, Tri- oder Tetraethylenglykoldi(meth)acrylat, Decandiol(meth)acrylat, Trimethylolpropantri(meth)acrylat, Pentaerythrittetra(meth)acrylat, Butandioldi(meth)acrylat, 1,10-Decandioldi(meth)acrylat oder 1,12-Dodecandiol-di(meth)acrylat oder eine Mischung dieser Monomere enthält.

9. Dentalmaterial nach einem der Ansprüche 1 bis 8, **dadurch gekennzeichnet, daß** es zusätzlich einen Füllstoff enthält.

10. Dentalmaterial nach einem der Ansprüche 1 bis 9, **dadurch gekennzeichnet, daß** es einen Initiator für die radikalische Polymerisation enthält.

11. Dentalmaterial nach einem der Ansprüche 1 bis 10, **dadurch gekennzeichnet, daß** es
(a) 5 bis 99,9 Gew.-% Polysiloxan; und
(b) 0,1 bis 5,0 Gew.-% Polymerisationsinitiator; und ggf.
(c) 1,0 bis 80 Gew.-% ionisch und/oder radikalisch polymerisierbares Monomer;
(d) 1,0 bis 90 Gew.-% Füllstoff enthält.

12. Verwendung eines Dentalmaterials gemäß einem der Ansprüche 1 bis 11 zur Herstellung eines Verbundmaterials, Zements, Füllungsmaterials oder Bondings.

## Claims

1. A dental material comprising at least one polysiloxane based on one or more silanes according to Formula (I)
[(W_{q}-R⁶-Z)ₚ -R³]ₘY-R²-SiXₙR¹ ₃₋ₙ Formula (I)
in which
X stands for a halogen atom, a hydroxyl group, an alkoxy group and/or acyloxy group;
n is equal to 1 to 3;
R¹ stands for an alkyl group, an alkenyl group, an aryl group, an alkylaryl group, an arylalkyl group;
R² stands for an alkylene group;
R³ stands for a p-times substituted, straight, branched or cyclic, saturated or unsaturated, aromatic or aliphatic organic radical with 2 to 40 carbon atoms and optionally 1 to 6 heteroatoms;
R⁶ stands for a q-times substituted, straight, branched or cyclic organic radical with 1 to 20 carbon atoms;
p is equal to 1 to 6;
q is equal to 1 to 6;
Y stands for -NR⁴-, N or -(C=O)-NH-;
m is equal to 2 for Y = N and equal to 1 for Y = -NR⁴- or -(C=O)-NH-;
R⁴ stands for an alkyl group or an aryl group;
Z stands for O, S, -(C=O)-O-, -(C=O)-NH-, -O-(C=O)-NH- or is absent;
W stands for CH₂=CR⁵-(C=O)-O-; and
R⁵ stands for a hydrogen atom or an alkyl group
and optionally one or more further hydrolytically condensable compounds of silicon, aluminium, zirconium, titanium, boron, tin, vanadium and/or phosphorus.

2. Dental material according to Claim 1, **characterised in that**
X stands for a methoxy group and/or an ethoxy group;
n is 2 or 3;
R¹ stands for a C₁ to C₃ alkyl group, particularly a methyl group;
R² stands for a C₁ to C₄ alkylene group;
R³ stands for a p-times substituted, straight, branched or cyclic, saturated or unsaturated, aromatic or aliphatic organic radical with 2 to 10 carbon atoms and optionally a heteroatom, preferably an oxygen atom, particularly for C₁ to C₄ alkenylene or a monocyclic radical with 4 to 10 carbon atoms;
R⁶ is a q-times substituted, straight, branched or cyclic organic radical with 1 to 4 carbon atoms, particularly a C₁ to C₃ alkylene radical;
p is equal to 1 or 2, particularly 1;
q is equal to 1 or 2;
Y stands for N or -(C=O)-NH-;
Z stands for -(C=O)-O-; and/or
R⁵ stands for a hydrogen atom or a methyl group.

3. Dental material according to Claim 1 or 2, **characterised in that** the further hydrolytically condensable compound is a silane according to Formula (II)
R⁷ ₖ(Z'R⁸)ₙSiX' ₄₋₍ₖ₊ₘ₎ Formula (II)
in which
R⁷ stands for a C₁ to C₈ alkyl, a C₂ to C₁₂ alkenyl or a C₆ to C₁₄ aryl group;
R⁸ stands for a C₁ to C₈ alkylene group, a C₂ to C₁₂ alkenylene group or C₆ to C₁₄ arylene group;
X' stands for a hydrogen atom, a halogen atom or a C₁ to C₈ alkoxy group;
Z' stands for a glycidyl group, an acrylic group, a methacrylic group, a vinyl group, an allyl group or vinyl ether group;
k is equal to 0, 1, 2 or 3;
m is equal to 0, 1, 2 or 3; and
k+m is equal to 0, 1, 2 or 3;
a zirconium compound, titanium compound of Formula (III)
MeX" _{y} R⁹ _{z} Formula (III)
in which
Me stands for Zr or Ti;
R⁹ stands for a hydrogen atom, a substituted or unsubstituted C₁ to C₁₂ alkyl, a C₁ to C₁₅ alkylaryl or C₆ to C₁₄ aryl group;
X" stands for a halogen atom, a hydroxyl group or a C₁ to C₈ alkoxy group;
Y equals 1 to 4; and
Z equals 1 to 3;
an aluminium compound according to Formula (IV)
Al R¹⁰ ₃ Formula (IV)
in which
R¹⁰ stands for a halogen atom, a hydroxyl or a C₁ to C₈ alkoxy group;
and/or a boron trihalide, tin tetrahalide, a tin tetraalkoxide and/or a vanadyl compound.

4. Dental material according to Claim 3, **characterised in that**
R⁷ is a C₁ to C₃ alkyl, C₂ to C₅ alkenyl or a phenyl group;
R⁸ is a C₁ to C₅ alkylene, C₂ to C₅ alkenylene or a phenylene group;
X' is a halogen atom, a methoxy group or an ethoxy group;
Z' is an acrylic group or a methacrylic group;
k is 0 or 1;
m is 0 or 1;
k+m is 0, 1 or 2
and/or
R⁹ is a C₁ to C₅ alkyl group or a phenyl group;
X" is a halogen atom, a methoxy group, an ethoxy group or a propoxy group;
Y is 4;
Z is 0 or 1, particularly 0;
and/or
R¹⁰ is a halogen atom or a C₁ to C₅ alkoxy group.

5. Dental material according to Claim 3 or 4, **characterised in that** it comprises
the silane or the silanes of the general Formula (II) or pre-condensed products derived therefrom in a quantity of 0 to 90 mol%, preferably 1 to 60 mol% and particularly 1 to 40 mol%; and/or
the zirconium compound and/or titanium compound of the general Formula (III) or pre-condensed products derived therefrom in a quantity of 0 to 70 mol%, preferably 0 to 50 mol% and particularly 0 to 30 mol%; and/or
the aluminum compound of the general Formula (IV) or pre-condensed products derived therefrom in a quantity of 0 to 70 mol%, preferably 0 to 30 mol% and particularly 0 to 20 mol%,
relative to the total weight of compounds of Formula (I) and/or pre-condensed products derived therefrom and compounds of Formula (II), Formula (III) or (IV).

6. Dental material according to one of Claims 1 to 5, **characterised in that** the polysiloxane comprises 10 to 99 mol% of a silane of Formula (I), based on the monomeric starting compound.

7. Dental material according to one of Claims 1 to 6, **characterised in that** it additionally comprises an ionically and/or radically polymerisable monomer.

8. Dental material according to Claim 7, **characterised in that** it comprises a methyl (meth)acrylate, ethyl (meth)acrylate, butyl (meth)acrylate, benzyl (meth)acrylate, furfuryl (meth)acrylate, phenyl (meth)acrylate, bisphenol-A-di(meth)acrylate, Bis-GMA, UDMA, diethyleneglycol di(meth)acrylate, triethyleneglycol di(meth)acrylate, tetraethyleneglycol di(meth)acrylate, decanediol (meth)acrylate, trimethylolpropane tri(meth)acrylate, pentaerythritol tetra(meth)acrylate, butanediol di(meth)acrylate, 1,10-decanediol di(meth)acrylate or 1,12-dodecanediol di(meth)acrylate or a mixture of these monomers.

9. Dental material according to one of Claims 1 to 8, **characterised in that** it further comprises a filler.

10. Dental material according to one of Claims 1 to 9, **characterised in that** it comprises an initiator for radical polymerisation.

11. Dental material according to on of Claims 1 to 10, **characterised in that** it comprises
(a) 5 to 99.9 wt.% polysiloxane; and
(b) 0.1 to 5.0 wt.% polymerisation initiator; and optionally
(c) 1.0 to 80 wt.% ionically and/or radically polymerisable monomer;
(d) 1.0 to 90 wt.% filler.

12. Use of a dental material according to one of Claims 1 to 11 in the manufacture of composite materials, cements, filling materials or bondings.

## Revendications

1. Matériau dentaire contenant au moins un polysiloxane à base d'un ou plusieurs silanes de formule (I)
[(W_{q}-R⁶-Z)ₚ-R³]ₘY-R²-SiXₙR¹ ₃₋ₙ Formule (I)
dans laquelle
X représente un atome d'halogène, un groupe hydroxy, alcoxy et/ou acyloxy ;
n a une valeur de 1 à 3 ;
R¹ représente un groupe alkyle, alcényle, aryle, alkylaryle, arylalkyle ;
R² représente un groupe alkylène ;
R³ représente un radical organique aromatique ou aliphatique à chaîne droite, ramifié ou cyclique, saturé ou insaturé, p-fois substitué, ayant de 2 à 40 atomes de carbone et comportant éventuellement 1 à 6 hétéroatomes ;
R⁶ représente un radical organique à chaîne droite, ramifié ou cyclique, q-fois substitué, ayant de 1 à 20 atomes de carbone ;
p a une valeur de 1 à 6 ;
q a une valeur de 1 à 6 ;
Y représente -NR⁴-, N ou -(C=O)-NH- ;
m est égal à 2 lorsque Y = N et est égal à 1 lorsque Y = -NR⁴- ou -(C=O)-NH- ;
R⁴ représente un groupe alkyle ou aryle ;
Z représente O, S, -(C=O)-O-, -(C=O)-NH-, -O-(C=O)-NH- ou est absent ;
W représente CH₂=CR⁵-(C=O)-O- ; et
R⁵ représente un atome d'hydrogène ou un groupe alkyle
et éventuellement d'un ou plusieurs autres composés, aptes à la condensation hydrolytique, du silicium, de l'aluminium, du zirconium, du titane, du bore, de l'étain, du vanadium et/ou du phosphore.

2. Matériau dentaire selon la revendication 1, **caractérisé en ce que**
X représente un groupe méthoxy et/ou un groupe éthoxy ;
n est 2 ou 3 ;
R¹ représente un groupe alkyle en C₁-C₃, en particulier un groupe méthyle ;
R² représente un groupe alkylène en C₁-C₄ ;
R³ représente un radical organique aromatique ou aliphatique à chaîne droite, ramifié ou cyclique, saturé ou insaturé, p-fois substitué, ayant de 2 à 10 atomes de carbone et comportant éventuellement un hétéroatome, de préférence un atome d'oxygène, en particulier un groupe alcénylène en C₁-C₄, ou un radical monocyclique ayant de 4 à 10 atomes de carbone ;
R⁶ représente un radical organique à chaîne droite, ramifié ou cyclique, q-fois substitué, ayant de 1 à 4 atomes de carbone, en particulier un radical alkylène en C₁-C₃ ;
p est égal à 1 ou 2, en particulier 1 ;
q est égal à 1 ou 2 ;
Y représente N ou -(C=O)-NH- ;
Z représente -(C=O)-O- ; et/ou
R⁵ représente un atome d'hydrogène ou un groupe méthyle.

3. Matériau dentaire selon la revendication 1 ou 2, **caractérisé en ce que** l'autre composé apte à la condensation hydrolytique est un silane de formule (II)
R⁷ ₖ(Z'R⁸)ₘSiX' ₄₋₍ₖ₊ₘ₎ Formule (II)
dans laquelle
R⁷ représente un groupe alkyle en C₁-C₈, alcényle en C₂-C₁₂ ou un groupe aryle en C₆-C₁₄ ;
R⁸ représente un groupe alkylène en C₁-C₈, alcénylène en C₂-C₁₂ ou arylène en C₆-C₁₄ ;
X' représente un atome d'hydrogène ou d'halogène ou un groupe alcoxy en C₁-C₈ ;
Z' représente un groupe éther glycidylique, acrylique, méthacrylique, vinylique, allylique ou vinylique ;
k est égal à 0, 1, 2 ou 3 ;
m est égal à 0, 1, 2 ou 3 ; et
k+m est égal à 0, 1, 2 ou 3 ;
un composé contenant du zirconium ou du titane de formule (III)
MeX" _{y}R⁹ _{z} formule (III)
dans laquelle
Me représente Zr ou Ti ;
R⁹ représente un atome d'hydrogène, un groupe alkyle en C₁-C₁₂, alkylaryle en C₁-C₁₅ ou aryle en C₆-C₁₄, substitué ou non substitué ;
X" représente un atome d'halogène, un groupe hydroxy ou alcoxy en C₁-C₈ ;
Y a une valeur de 1 à 4 ; et
Z a une valeur de 1 à 3 ;
un composé d'aluminium de formule IV
Al-R¹⁰ ₃ Formule (IV)
dans laquelle
R¹⁰ représente un atome d'halogène, un groupe hydroxy ou alcoxy en C₁-C₈ ;
et/ou un trihalogénure de bore, tétrahalogénure d'étain, tétra-alcoolate d'étain et/ou un composé vanadyle.

4. Matériau dentaire selon la revendication 3, **caractérisé en ce que**
R⁷ est un groupe alkyle en C₁-C₃, alcényle en C₂-C₅ ou un groupe phényle ;
R⁸ est un groupe alkylène en C₁-C₅, alcénylène en C₂-C₅ ou un groupe phénylène ;
X' est un atome d'halogène, un groupe méthoxy ou éthoxy ;
Z' est un groupe acryloyle ou méthacryloyle ;
k est 0 ou 1 ;
m est 0 ou 1 ;
k+m est 0, 1 ou 2 ;
et/ou
R⁹ est un groupe alkyle en C₁-C₅ ou un groupe phényle ;
X" est un atome d'halogène, un groupe méthoxy, éthoxy ou propoxy ;
Y = 4;
Z est 0 ou 1, en particulier 0 ;
et/ou
R¹⁰ est un atome d'halogène ou un groupe alcoxy en C₁-C₅.

5. Matériau dentaire selon la revendication 3 ou 4, **caractérisé en ce qu'**il contient
le ou les silanes de formule générale (II) ou des produits précondensés dérivés de ceux-ci en une proportion de 1 à 90 % en moles, de préférence de 1 à 60 % en moles et en particulier de 1 à 40 % en moles ; et/ou
le composé de zirconium ou de titane de formule générale (III) ou des produits précondensés dérivés de celui-ci en une proportion de 0 à 70 % en moles, de préférence de 0 à 50 % en moles et en particulier de 0 à 30 % en moles ; et/ou
le composé d'aluminium de formule générale (IV) ou des produits précondensés dérivés de celui-ci en une proportion de 0 à 70 % en moles, de préférence de 0 à 30 % en moles et en particulier de 0 à 20 % en moles,
par rapport à la masse totale des composés de formule (I) et/ou des produits précondensés dérivés de ceux-ci et des composés de formule (II), de formule (III) ou (IV), respectivement.

6. Matériau dentaire selon l'une quelconque des revendications 1 à 5, **caractérisé en ce que** le polysiloxane contient de 10 à 99 % en moles d'un silane de formule (I), par rapport aux composés monomères de départ.

7. Matériau dentaire selon l'une quelconque des revendications 1 à 6, **caractérisé en ce qu'**il contient en outre un monomère polymérisable par voie ionique et/ou radicalaire.

8. Matériau dentaire selon la revendication 7, **caractérisé en ce qu'**il contient un monomère (méth)acrylate de méthyle, éthyle, butyle, benzyle, furfuryle et/ou phényle, di(méth)acrylate de bisphénol A, bis-GMA, UDMA, di(méth)acrylate de di-, tri- ou tétra-éthylèneglycol, (méth)acrylate de décanediol, tri(méth)acrylate de triméthylolpropane, tétra(méth)acrylate de pentaérythritol, di(méth)acrylate de butanediol, di(méth)acrylate de 1,10-décanediol ou di(méth)acrylate de 1,12-dodécanediol ou un mélange de ces monomères.

9. Matériau dentaire selon l'une quelconque des revendications 1 à 8, **caractérisé en ce qu'**il contient en outre une charge.

10. Matériau dentaire selon l'une quelconque des revendications 1 à 9, **caractérisé en ce qu'**il contient un amorceur pour la polymérisation radicalaire.

11. Matériau dentaire selon l'une quelconque des revendications 1 à 10, **caractérisé en ce qu'**il contient
(a) 5 à 99,9 % en poids de polysiloxane ; et
(b) 0,1 à 5,0 % en poids d'amorceur de polymérisation ; et éventuellement
(c) 1,0 à 80 % en poids de monomère apte à la polymérisation ionique et/ou radicalaire ;
(d) 1,0 à 90 % en poids de charge.

12. Utilisation d'un matériau dentaire selon l'une quelconque des revendications 1 à 11, pour la préparation d'un matériau composite, d'une colle, d'une matière d'obturation ou d'un agent de collage.
